(19) Europäisches Patentamt
European Patent Office
Office européen des brevets

(11) EP 0 716 680 B1

# (12) EUROPÄISCHE PATENTSCHRIFT

(45) Veröffentlichungstag und Bekanntmachung des Hinweises auf die Patenterteilung:
**11.02.1998 Patentblatt 1998/07**

(21) Anmeldenummer: **94925471.8**

(22) Anmeldetag: **24.08.1994**

(51) Int Cl.[6]: **C11D 1/825**, C11D 1/83, A61K 7/50, A61L 2/18

(86) Internationale Anmeldenummer:
**PCT/EP94/02798**

(87) Internationale Veröffentlichungsnummer:
**WO 95/06702 (09.03.1995 Gazette 1995/11)**

## (54) VERWENDUNG WÄSSRIGER DETERGENSGEMISCHE

USE OF AQUEOUS DETERGENT MIXTURES

UTILISATION DE MELANGES DETERGENTS AQUEUX

(84) Benannte Vertragsstaaten:
**AT BE DE ES FR GB IT NL**

(30) Priorität: **02.09.1993 US 116658**

(43) Veröffentlichungstag der Anmeldung:
**19.06.1996 Patentblatt 1996/25**

(73) Patentinhaber: **Henkel Kommanditgesellschaft auf Aktien**
**40191 Düsseldorf (DE)**

(72) Erfinder:
- **BEHLER, Ansgar**
  **D-46230 Bottrop (DE)**
- **GIESEN, Brigitte**
  **D-40235 Düsseldorf (DE)**
- **RATHS, Hans-Christian**
  **D-40789 Monheim (DE)**
- **WULFF, Harald**
  **Bryn Mawr, PA 19010 (US)**

(56) Entgegenhaltungen:
**WO-A-91/14760** **WO-A-92/21742**
**WO-A-94/18291** **WO-A-95/05798**

- **PATENT ABSTRACTS OF JAPAN vol. 016 no. 387 (C-0975) ,18.August 1992 & JP,A,04 126799 (KANEBO LTD) 27.April 1992,**
- **DATABASE WPI Section Ch, Week 9320 Derwent Publications Ltd., London, GB; Class A97, AN 93-164787 & JP,A,05 098 289 (KAO CORP) , 20.April 1993**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 716 680 B1

## Beschreibung

### Gebiet der Erfindung

Gegenstand der Erfindung ist die Verwendung wäßriger Detergensgemische, enthaltend Alkyl- und/ oder Alkenyloligoglykoside und Monoglycerid(ether)sulfate zur Herstellung ausgewählter oberflächenaktiver Mittel.

### Stand der Technik

Alkyloligoglykoside und insbesondere Alkyloligoglucoside stellen nichtionische Tenside dar, die wegen ihrer nativen Rohstoffbasis - Fettalkohol und Zucker - zunehmend an Bedeutung gewinnen und beispielsweise in manuellen Spülmitteln oder kosmetischen Produkten eingesetzt werden [vgl. **Tens.Surf. Det. 28, 413 (1991)]**. Trotz guter anwendungstechnischer Ergebnisse besteht dennoch das Bedürfnis nach Detergensmischungen auf Basis von Alkylglucosiden, deren Leistungsvermögen das der Einzelstoffe in synergistischer Weise übersteigt.

In der Vergangenheit hat es nicht an Versuchen gemangelt, Detergensgemische auf Basis von Alkyloligoglucosiden zu entwickeln, die über vorteilhafte Eigenschaften verfügen. Aus den Patentschriften **EP-B1 0070074, EP-B1 0070075, EP-B1 070076** und **EP-B1 0070077** (Procter & Gamble) sind beispielsweise schaumstarke Kombinationen von Alkyloligoglucosiden mit anionischen Tensiden, wie Seifen, Alkylbenzolsulfonaten, Fettalkoholsulfaten, konventionellen Fettalkoholethersulfaten, α-Olefinsulfonaten und Alkansulfonaten bekannt. Kombinationen von Alkyloligoglucosiden und Fettalkoholpolyglycolethem konventioneller Homologenverteilung sind beispielsweise aus der **EP-B1 0075 995** und **EP-B1 0075996** (Procter & Gamble), der **EP-A 0317614** (Staley) sowie der **WO 91/03538** (Henkel) bekannt. Gegenstand der beiden älteren Rechte **WO 95/05798** und **WO 94/18291** sind Rasiermittel mit einem Gehalt an Alkyloligoglucosiden und Monoglyceridsulfaten sowie feste Waschmittel, die u.a. Alkyloligoglucoside und Monoglyceridsulfate enthalten können.

Bei Einsatz dieser bekannten Detergensmischungen in oberflächenaktiven Mitteln müssen jedoch bislang in manchen Fällen Abstnche hinsichtlich des Leistungsvermögens und der ökotoxikologischen Verträglichkeit in Kauf genommen werden.

Die Aufgabe der Erfindung bestand somit darin, Detergensgemische auf der Basis von Alkyl- und/oder Alkenyloligoglykosiden mit weiter verbesserten Eigenschaften zu entwickeln.

### Beschreibung der Erfindung

Gegenstand der Erfindung ist die Verwendung wäßriger Detergensgemische, enthaltend

(a) Alkyl- und/oder Alkenyloligoglykoside und
(b) Monoglycerid(ether)sulfate

zur Herstellung von flüssigen Wasch-, Spül- und Reinigungsmitteln, Haarpflegemitteln und Schaumbädem.

Überraschenderweise wurde gefunden, daß die erfindungsgemäßen Detergensgemische über ein Wasch-, Spül-, Schaum- und Reinigungsvermögen sowie eine hautkosmetische Verträglichkeit verfügen, die die der Einzelstoffe im Sinne einer synergistischen Verstärkung übertreffen.

#### Alkyl- und/oder Alkenyloligoglykoside

Alkyl- und/oder Alkenyloligoglykoside, die die Komponente (a) bilden, stellen bekannte Stoffe dar, die nach den einschlägigen Verfahren der präparativen organischen Chemie erhalten werden können. Stellvertretend für das umfangreiche Schrifttum sei hier auf die Schriften **EP-A1 0301298** und **WO 90/3977** verwiesen. Die Alkyl- und/oder Alkenyloligoglykoside folgen der Formel (I),

$$R^1O\text{-}[G]_p \qquad (I)$$

in der $R^1$ für einen linearen oder verzweigten Alkyl- oder Alkenylrest mit 4 bis 22 Kohlenstoffatomen, [G] für eine Glykoseeinheit mit 5 oder 6 Kohlenstoffatomen und p für eine Zahl von 1 bis 10 steht. Die Alkyl- und/oder Alkenyloligoglykoside können sich von Aldosen bzw. Ketosen mit 5 oder 6 Kohlenstoffatomen, vorzugsweise der Glucose ableiten. Die bevorzugten Alkyl- und/oder Alkenyloligoglykoside sind somit Alkyl- und/oder Alkenyloligoglucoside. Die Indexzahl p in der allgemeinen Formel (I) gibt den Oligomerisierungsgrad (DP), d. h. die Verteilung von Mono- und Oligoglykosiden an und steht für eine Zahl zwischen 1 und 10. Während p in einer gegebenen Verbindung stets ganzzahlig sein muß und hier vor allem die Werte p = 1 bis 6 annehmen kann, ist der Wert p für ein bestimmtes Alkyloligoglykosid eine analytisch ermittelte rechnerische Größe, die meistens eine gebrochene Zahl darstellt. Vorzugsweise werden Alkyl- und/oder Alkenyloligoglykoside mit einem mittleren Oligomerisierungsgrad p von 1,1 bis 3,0 eingesetzt. Aus anwendungstechnischer Sicht sind solche Alkyl- und/oder Alkenyloligoglykoside bevorzugt, deren Oligomerisierungsgrad kleiner als 1,7 ist und insbesondere zwischen 1,2 und 1,4 liegt.

Der Alkyl- bzw. Alkenylrest $R^1$ kann sich von primären Alkoholen mit 4 bis 11, vorzugsweise 8 bis 10 Kohlenstoffatomen ableiten. Typische Beispiele sind Butanol, Capronalkohol, Caprylalkohol, Caprinalkohol und Undecylalkohol sowie deren technische Mischungen, wie sie beispielsweise bei der Hydrierung von technischen Fettsäuremethylestem oder im Verlauf der Hydrierung von Aldehyden aus der Roelen'schen Oxosynthese anfallen. Bevorzugt sind Alkyloligoglucoside der Kettenlänge $C_8$-$C_{10}$ (DP = 1 bis 3), die als Vorlauf bei der destillativen Auftrennung von technischem $C_8$-$C_{18}$-Kokosfettalkohol anfallen und mit einem Anteil von weniger als 6 Gew.-% $C_{12}$ Alkohol verunreinigt sein können sowie Alkyloligoglucoside auf Basis technischer $C_{9/11}$-Oxoalkohole (DP = 1 bis 3). Der Alkyl- bzw. Alkenylrest $R^1$ kann sich femer auch von primären Alkoholen mit 12 bis 22, vorzugsweise 12 bis 14 Kohlenstoffatomen ableiten. Typische Beispiele sind Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoieylalkohol, Behenylalkohol, Erucylalkohol, sowie deren technische Gemische, die wie oben beschrieben erhalten werden können. Bevorzugt sind Alkyloligoglucoside auf Basis von gehärtetem $C_{12/14}$-Kokosalkohol mit einem DP von 1 bis 3.

<u>Monoglycerid(ether)sulfate</u>

Die im Sinne der Erfindung als Komponente (b) einzusetzenden Monoglyceid(ether)sulfate folgen der Formel **(II)**,

$$\begin{array}{l} CH_2O(CH_2CH_2O)_x\text{-}COR^2 \\ \quad | \\ CH\text{-}O(CH_2CH_2O)_yH \\ \quad | \\ CH_2O(CH_2CH_2O)_z\text{-}SO3X \end{array} \qquad \textbf{(II)}$$

in der $R^2CO$ für einen linearen oder verzweigten Acylrest mit 6 bis 22 Kohlenstoffatomen, x, y und z in Summe für 0 oder für Zahlen von 1 bis 30 und X für ein Alkali- oder Erdalkalimetall steht. Auch bei diesen Stoffen handelt es sich um bekannte chemische Verbindungen, die man beispielsweise durch Sulfatierung von Partialglyceriden oder deren Ethylenoxidaddukten und nachfolgende Neutralisation erhalten kann [vgl. **WO 92/09569**, **WO 92/09570,** Henkel]. Typische Beispiele sind die Umsetzungsprodukte von Laurinsäuremonoglycerid, Kokosfettsäuremonoglycerid, Palmitinsäuremonoglycerid, Stearinsäuremonoglycerid, Ölsäuremonoglycerid und Talgfettsäuremonoglycerid sowie deren Ethylenoxidaddukten mit Schwefeltrioxid oder Chlorsulfonsäure in Form ihrer Natriumsalze. Vorzugsweise werden Monoglyceridsulfate der Formel **(II)** eingesetzt, in der $R^2CO$ für einen linearen Acylrest mit 8 bis 18 Kohlenstoffatomen steht.

<u>Detergensgemische</u>

Die erfindungsgemäßen wäßrigen Detergensgemische können die Alkyl- und/oder Alkenyloligoglykoside in Mengen von 1 bis 99, vorzugsweise 30 bis 75 Gew.-% - bezogen auf den Feststoffanteil der Gemische - enthalten. Der Anteil der Monoglycerid(ether)sulfate kann 1 bis 75, vorzugsweise 10 bis 70 und insbesondere 25 bis 60 Gew.-% betragen. Die Herstellung der wäßigen Detergensgemische kann durch einfaches mechanisches Vermischen der wäßrigen Lösungen der Komponenten, gegebenenfalls bei erhöhten Temperaturen von 30 bis 50°C erfolgen; eine chemische Reaktion findet hierbei nicht statt.

Im folgenden werden weitere Gegenstände beschrieben, die die Erfindung beispielhaft beschreiben:

0 **Pulverförmige Universalwaschmittel,** enthaltend 10 bis 30 Gew.-% - bezogen auf das Mittel - einer Mischung von Alkyl- und/oder Alkenyloligoglykosiden und Monoglycerid(ether)sulfaten sowie gegebenenfalls weitere übliche Hilfs- und Zusatzstoffe.

0 **Flüssige Universalwaschmittel,** enthaltend 10 bis 70 Gew.-% - bezogen auf das Mittel - einer Mischung von Alkyl- und/oder Alkenyloligoglykosiden und Monoglycerid(ether)sulfaten sowie gegebenenfalls weitere übliche Hilfs- und Zusatzstoffe.

- 0 **Flüssige Feinwaschmittel,** enthaltend 10 bis 50 Gew.-% - bezogen auf das Mittel - einer Mischung von Alkyl- und/oder Alkenyloligoglykosiden und Monoglycerid(ether)sulfaten sowie gegebenenfalls weitere übliche Hilfs- und Zusatzstoffe.

- 0 **Flüssige Reinigungs- und Desinfektionsmittel,** enthaltend 10 bis 30 Gew.-%- bezogen auf das Mittel - einer Mischung von Alkyl- und/oder Alkenyloligoglykosiden und Monoglycerid(ether)sulfaten sowie gegebenenfalls weitere übliche Hilfs- und Zusatzstoffe.

- 0 **Haarshampoos,** enthaltend 10 bis 30 Gew.-% - bezogen auf das Mittel - einer Mischung von Alkyl- und/oder Alkenyloligoglykosiden und Monoglycerid(ether)sulfaten sowie gegebenenfalls weitere übliche Hilfs- und Zusatzstoffe.

- 0 **Haarspülungen,** enthaltend 10 bis 30 Gew.-% - bezogen auf das Mittel - einer Mischung von Alkyl- und/oder Alkenyloligoglykosiden und Monoglycerid(ether)sulfaten sowie gegebenenfalls weitere übliche Hilfs- und Zusatzstoffe.

- 0 **Schaumbäder,** enthaltend 10 bis 30 Gew.-% bezogen auf das Mittel - einer Mischung von Alkyl-und/ oder Alkenyloligoglykosiden und Monoglycerid(ether)sulfaten sowie gegebenenfalls weitere übliche Hilfs- und Zusatzstoffe.

<u>Wasch- und Reinigungsmittel</u>

Wasch- und Reinigungsmittel auf Basis der erfindungsgemäßen Detergensgemische können als Hilfs- und Zusatzstoffe beispielsweise Builder, Salze, Bleichmittel, Bleichaktivatoren, optische Aufheller, Vergrauungsinhibitoren, Lösungsvermittler, Entschäumer und Enzyme enthalten. Übliche **Builder** sind Natriumaluminiumsilicate (Zeolithe), Phosphate, Phosphonate, Ethylendiamintetraessigsäure, Nitrilotriacetat, Citronensäure und/oder Polycarboxylate. Als **Salze** bzw. Stellmittel kommen beispielsweise Natriumsulfat, Natriumcarbonat oder Natriumsilicat (Wasserglas) in Betracht. Als typische Einzelbeispiele für weitere Zusatzstoffe sind Natriumborat, Stärke, Saccharose, Polydextrose, TAED, Stilbenverbindungen, Methylcellulose, Toluolsulfonat, Cumolsulfonat, langkettige Seifen, Silicone, Mischether, Lipasen und Proteasen zu nennen.

<u>Haarbehandlungsmittel und Schaumbäder</u>

Haarshampoos, Haarlotionen oder Schaumbäder auf Basis der erfindungsgemäßen Detergensgemische können als Hilfs- und Zusatzstoffe beispielsweise Emulgatoren, Ölkomponenten, Fette und Wachse, Verdickungsmittel, Überfettungsmittel, biogene Wirkstoffe, Filmbildner, Duftstoffe, Farbstoffe, Perlglanzmittel, Konservierungsmittel und pH-Regulatoren enthalten. Übliche **Ölkomponenten** sind Substanzen wie Paraffinöl, Pflanzenöle, Fettsäureester, Squalan und 2-Octyldodecanol, während als **Fette und Wachse** beispielsweise Walrat, Bienenwachs, Montanwachs, Paraffin und Cetylstearylalkohol Verwendung finden. Als **Überfettungsmittel** können Substanzen wie beispielsweise polyethoxylierte Lanolinderivate, Lecithinderivate und Fettsäurealkanolamide verwendet werden, wobei die letzteren gleichzeitig als Schaumstabilisatoren dienen. Geeignete **Verdickungsmittel** sind beispielsweise Polysaccharide, insbesondere Xanthan-Gum, Guar-Guar, Agar-Agar, Alginate und Tylosen, Carboxymethylcellulose und Hydroxyethylcellulose, femer höhermolekulare Polyethylenglycolmonound -diester von Fettsäuren, Polyacrylate, Polyvinylalkohol und Polyvinylpyrrolidon sowie Elektrolyte wie Kochsalz und Ammoniumchlorid. Unter **biogenen Wirkstoffen** sind beispielsweise Pflanzenextrakte, Eiweißhydrolysate und Vitaminkomplexe zu verstehen. Gebräuchliche Filmbildner sind beispielsweise Polyvinylpyrrolidon, Vinylpyrrolidon-Vinylacetat-Copolymerisate, Polymere der Acrylsäurereihe, quatemäre Cellulose-Derivate und ähnliche Verbindungen. Als **Konservierungsmittel** eignen sich beispielsweise Formaldehydlösung, p-Hydroxybenzoat oder Sorbinsäure. Als **Perlglanzmittel** kommen beispielsweise Glycoldistearinsäureester wie Ethylenglycoldistearat, aber auch Fettsäuremonoglycolester in Betracht. Als **Farbstoffe** können die für kosmetische Zwecke geeigneten und zugelassenen Substanzen verwendet werden, wie sie beispielsweise in der Publikation **"Kosmetische Färbemittel" der Farbstoffkommission der Deutschen Forschungsgemeinschaft, veröffentlicht im Verlag Chemie, Weinheim, 1984,** zusammengestellt sind. Diese Farbstoffe werden üblicherweise in Konzentrationen von 0,001 bis 0,1 Gew.-%, bezogen auf die gesamte Mischung, eingesetzt.

## <u>Beispiele</u>

**Waschvermögen**. Die Untersuchung des Waschvermögens wurde in einem Launderometer an einer Staub-Hautfett-Anschmutzung auf Polyester/Baumwoll (veredelt) Gewebe bei 40°C durchgeführt. Die Beurteilung der Aufhellung des gewaschenen Gewebes erfolgte durch photometrische Remissionsmessung mit einem Elrepho RFC-3/24-Gerät

gegenüber einem Bariumsulfat-Standard, dessen Remission zu 100 % gesetzt wurde. Die Angabe erfolgt in %-Remission (%-R). Es galten folgende Bedingungen: Flotte : 250 ml; Flottenbelastung : 1 Gew.-Teil Gewebe/30 Gew.-% Teile Wasser; Dosierung : 10 g/l; Wasserhärte : 16°d; Bestimmung : Mittel von 3 Bestimmungen.

**Tellerspülvermögen**. Die Ermittlung des Tellerspülvermögens wurde mit Hilfe des Teller-Testes **[Fette, Seifen, Anstrichmitt.**, **74**, **163 (1972)]** durchgeführt. Hierzu wurden Teller mit einem Durchmesser von 14 cm mit je 2 ml Rindertalg (Säurezahl 9-10) angeschmutzt und 24 h bei Raumtemperatur gelagert. Anschließend wurden die Teller bei 50°C mit 5 l Leitungswasser der Härte 16°d gespült. Die Prüfmischung wurde mit einer Dosierung von 0,15 g Aktivsubstanz/l eingesetzt. Der Spülversuch wurde abgebrochen, sobald der Schaum an der Oberfläche völlig verschwunden war.

**Basisschaumvermögen.** Die Beurteilung des Schaumvermögens wurde gemäß **DIN 53 902** nach der Götte-Schlagschaum-Methode durchgeführt. Es wurden 1 Gew.-%ige Tensidlösungen in Wasser von 16° Härte eingesetzt; die Temperatur betrug 20°C.

Die Ergebnisse sind in Tabelle 1 zusammengefaßt. Die Beispiele 1 bis 3 sind erfindungsgemäß, die Beispiele V1 bis V5 dienen zum Vergleich. Die Mengenangaben verstehen sich als Gewichtsteile.

**Tabelle 1:**
**Anwendungstechnische Untersuchungen**

| Zusammensetzung / Performance | 1 | 2 | 3 | V1 | V2 | V3 | V4 | V5 |
|---|---|---|---|---|---|---|---|---|
| $C_{12/14}$-Kokosalkyloligoglucosid | 75 | 75 | - | - | 75 | 75 | 75 | 75 |
| $C_{8/10}$-Alkyloligoglucosid | - | - | 75 | - | - | - | - | - |
| $C_{8/18}$-Kokosmonoglyceridsulfat-Natriumsalz | 25 | - | 25 | 100 | - | - | - | - |
| $C_{12/14}$-Kokosmonoglycerid+5EO-sulfat-Natriumsalz | - | 25 | - | - | - | - | - | - |
| Dodecylbenzolsulfonat-Natriumsalz | - | - | - | - | 25 | - | - | - |
| $C_{16/18}$-Talgfettalkoholsulfat-Natriumsalz | - | - | - | - | - | 25 | - | - |
| $C_{12/14}$-Kokosfettalkohol+3,6EO-sulfat-Natriumsalz | - | - | - | - | - | - | 25 | - |
| $C_{12/14}$-Kokosfettalkohol+2,5 EO | - | - | - | - | - | - | - | 25 |
| *Waschvermögen [%-R]* | 63,5 | 63,8 | 60,4 | 59,3 | 61,4 | 62,0 | 61,8 | 62,0 |
| *Tellerspülvermögen [Anzahl]* | 10 | 10 | 10 | 5 | 11 | 5 | 11 | 0 |
| *Schaumvermögen [ml]* | 370 | 380 | 310 | 300 | 390 | 370 | 390 | 0 |

## Patentansprüche

1. Verwendung wäßriger Detergensgemische, enthaltend

   (a) Alkyl- und/oder Alkenyloligoglykoside und
   (b) Monoglycerid(ether)sulfate

   zur Herstellung von flüssigen Wasch, Spül- und Reinigungsmitteln.

2. Verwendung von Detergensgemischen nach Anspruch 1 zur Herstellung von Haarpflegemitteln.

3. Verwendung von Detergensgemischen nach Anspruch 1 zur Herstellung von Schaumbädem.

4. Verwendung von Detergensgemischen nach den Ansprüchen 1 bis 3, **dadurch gekennzeichnet,** daß man Alkyl- und/oder Alkenyloligoglykoside der Formel **(I)** einsetzt,

$$R^1O\text{-}[G]_p \qquad (I)$$

in der $R^1$ für einen linearen oder verzweigten Alkyl- oder Alkenylrest mit 4 bis 22 Kohlenstoffatomen, [G] für eine Glykoseeinheit mit 5 oder 6 Kohlenstoffatomen und p für eine Zahl von 1 bis 10 steht.

**5.** Verwendung von Detergensgemischen nach den Ansprüchen 1 bis 4, dadurch gekennzeichnet, daß man Monoglycerid(ether)sulfate der Formel **(II)** einsetzt,

$$\begin{array}{l} CH_2O(CH_2CH_2O)_xCOR^2 \\ | \\ CH\text{-}O(CH_2CH_2O)_yH \\ | \\ CH_2O(CH_2CH_2O)_z\text{-}SO_3X \end{array} \qquad (II)$$

in der $R^2CO$ für einen linearen oder verzweigten Acylrest mit 6 bis 22 Kohlenstoffatomen, x, y und z in Summe für 0 oder für Zahlen von 1 bis 30 und X für ein Alkali- oder Erdalkalimetall steht.

**Claims**

**1.** The use of aqueous detergent mixtures containing

a) alkyl and/or alkenyl oligoglycosides and
b) monoglyceride (ether) sulfates

for the production of liquid laundry detergents, dishwashing detergents and cleaners.

**2.** The use of detergent mixtures according to claim 1 for the production of hair-care formulations.

**3.** The use of detergent mixtures according to the claim 1 for the production of foam baths.

**4.** The use of detergent mixtures according to claims 1 to 3, characterized in that alkyl and/or alkenyl oligoglycosides corresponding to formula **(I)**:

$$R^1O\text{-}[G]_p \qquad (I)$$

in which $R^1$ is a linear or branched alkyl or alkenyl radical containing 4 to 22 carbon atoms, [G] is a glycose unit containing 5 or 6 carbon atoms and p is a number of 1 to 10, are used.

**5.** The use of detergent mixtures according to claims 1 to 4, characterized in that monoglyceride (ether) sulfates corresponding to formula **(II)**:

$$\begin{array}{l} CH_2O(CH_2CH_2O)_x\text{-}COR^2 \\ | \\ CH\text{-}O(CH_2CH_2O)_y\text{-}H \\ | \\ CH_2O(CH_2CH_2O)_z\text{-}SO_3X \end{array} \qquad (II)$$

in which $R^2CO$ is a linear or branched acyl radical containing 6 to 22 carbon atoms, x, y and z together total 0 or

represent numbers of 1 to 30 and X is an alkali metal or alkaline earth metal.

**Revendications**

1. Utilisation de mélanges aqueux de détergents contenant :

   a) des alkyl- et/ou alkényloligoglycosides et,
   b) des sulfates (d'éther) de monoglycérides

   pour la fabrication de produits de lavage, de rinçage et de nettoyage liquides.

2. Utilisation de mélanges de détergents selon la revendication 1, pour la fabrication de produits de soins capillaires.

3. Utilisation de mélanges de détergents selon la revendication 1, pour la fabrication de bains moussants.

4. Utilisation de mélanges de détergents selon les revendications 1 à 3, caractérisée en ce qu' on met en oeuvre des alkyl- et/ou alkényloligoglycosides de formule (I),

$$R^1\text{-}O(G)_p \qquad (I)$$

   dans laquelle $R^1$ représente un radical alkyle ou alkényle linéaire ou ramifié, ayant de 4 à 22 atomes de carbone, (G) représente une unité de glycose ayant 5 ou 6 atomes de carbone et p représente un nombre allant de 1 à 10.

5. Utilisation de mélanges de détergents selon les revendications 1 à 4, caractérisé en ce qu' on met en oeuvre des sulfates (d'éther) de monoglycéride de formule (II),

$$\begin{array}{l} CH_2O(CH_2CH_2O)_xCOR^2 \\ | \\ CH\text{-}O(CH_2CH_2O)_yH \\ | \\ CH_2O(CH_2CH_2O)_z\text{-}SO_3X \end{array} \qquad (II)$$

   dans laquelle $R^2CO$ représente un radical acyle linéaire ou ramifié ayant de 6 à 22 atomes de carbone,

   x, y et z globalement représentent O ou des nombres allant de 1 à 30 et,
   X représente un métal alcalin ou alcalino-terreux.